(19) Europäisches Patentamt
European Patent Office
Office européen des brevets

(11) Veröffentlichungsnummer: **0 255 673 B1**

(12) **EUROPÄISCHE PATENTSCHRIFT**

(45) Veröffentlichungstag der Patentschrift: **06.11.91**

(51) Int. Cl.$^5$: **B01J 38/64**, B01J 31/40, C01G 55/00, C22B 3/32

(21) Anmeldenummer: **87110815.5**

(22) Anmeldetag: **25.07.87**

# (54) Verfahren zur Rückgewinnung von Rhodium aus Rhodiumkomplex-verbindungen enthaltenden wässrigen Lösungen.

(30) Priorität: **06.08.86 DE 3626536**

(43) Veröffentlichungstag der Anmeldung:
**10.02.88 Patentblatt 88/06**

(45) Bekanntmachung des Hinweises auf die Patenterteilung:
**06.11.91 Patentblatt 91/45**

(84) Benannte Vertragsstaaten:
**AT BE CH DE ES FR GB IT LI LU NL SE**

(56) Entgegenhaltungen:
**EP-A- 0 103 845**
**FR-A- 2 314 910**
**GB-A- 1 283 389**
**US-A- 3 251 646**
**US-A- 3 547 964**
**US-A- 4 390 473**

(73) Patentinhaber: **HOECHST AKTIENGESELLSCHAFT**
**Postfach 80 03 20**
**W-6230 Frankfurt am Main 80(DE)**

(72) Erfinder: **Bexten, Ludger, Dr. Dipl.-Chem.**
**Im Freihof 9**
**W-4224 Hünxe(DE)**
Erfinder: **Kupies, Dieter**
**Burgfeld 143**
**W-4100 Duisburg 14(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft ein Verfahren zur Rückgewinnung von Rhodium aus Rhodiumkomplexverbindungen enthaltenden wäßrigen Lösungen. Die Rhodiumkomplexverbindungen bilden zusammen mit überschüssig eingesetzten Komplexliganden unter anderem ein Katalysatorsystem, das - wie in der DE-PS 26 27 354 beschrieben - zur Hydroformylierung von Olefinen eingesetzt wird. Das Katalysatorsystem bildet sich unter Reaktionsbedingungen aus Rhodium und einem wasserlöslichen organischen Phosphin, das im Überschuß eingesetzt wird. Seine Wasserlöslichkeit beruht auf der Anwesenheit von Sulfonsäuregruppen, die in dem organischen Phosphin vorhanden sind. Der Phosphor-Ligand wird bevorzugt als Alkali-, Ammonium- oder Erdalkalisulfonat verwendet.

Bei längerem Gebrauch des Katalysatorsystems nimmt die Selektivität der Umsetzung ab. Diese Selektivitätsminderung ist zum einen auf das Einwirken von Katalysatorgiften, wie Eisencarbonyl, das sich durch Einwirken von Kohlenmonoxid auf die Wand des Reaktors bilden kann, und auf höhersiedende, sich aus den Aldehyden bildende Kondensationsprodukte und zum anderen auf die Verringerung des überschüssig eingesetzten sulfonierten Phosphins durch Oxidation zu Phosphinoxiden oder Abbau zu aromatischen Sulfonsäuren zurückzuführen. Daneben entstehen aus den Phosphinen und den im Synthesegas enthaltenen Schwefelverbindungen wie auch durch Reduktion von Sulfonsäuregruppen auch Phosphinsulfide.

Da weder Phosphinoxide noch Phosphinsulfide noch aromatische Sulfonsäuren im Hydroformylierungskatalysator erwünscht sind, ist es erforderlich, die verbrauchte Katalysatorlösung zu ersetzen. Aus Gründen der Wirtschaftlichkeit ist es notwendig, aus dieser Katalysatorlösung das Rhodium abzutrennen und zurückzugewinnen.

Die DE 32 35 029-Al betrifft ein Verfahren zur Rückgewinnung von Katalysatorsystemen, die gemäß DE-PS 26 27 354 zur Hydroformylierung verwendet werden. Man setzt durch Ansäuern aus den Sulfonsäuresalzen die Sulfonsäuren frei, extrahiert diese mit einem in einem organischen Lösungsmittel gelösten Amin, trennt die organische Phase, die nunmehr das Aminsalz des sulfonierten Phosphins enthält, von der wäßrigen Lösung ab, behandelt sie mit wäßriger Lauge und separiert die beiden sich bildenden Phasen. Die wäßrige Phase enthält nunmehr die Rhodiumkomplexverbindung und das Phosphinsulfonat.

Eine gezielte Rückgewinnung des Rhodiums aus der Rhodiumkomplexverbindung ist nur in begrenztem Umfang möglich, da die Rhodiumkomplexverbindung stets in unveränderter Form zusammen mit überschüssig eingesetztem Liganden erhalten wird.

Die US-PS 43 90 473 betrifft ein Verfahren zur Rückgewinnung von Rhodium und Kobalt aus der Lösung eines in einem organischen Solvens gelösten Hydroformylierungskatalysators, in der neben Rhodium- und Kobaltkomplexverbindungen noch Triphenylphosphin als Ligand vorhanden ist. Diese Lösung wird mit wäßriger Ameisensäure versetzt und anschließend mittels eines sauerstoffhaltigen Gases oxidiert. Dabei werden die Rhodium- und Kobaltkomplexverbindungen gespalten und überschüssiges Triphenylphosphin oxidiert. Rhodium und Kobalt werden als Formiate in die wäßrige Phase überführt und von der organischen Lösung abgetrennt. Eine Rückgewinnung von Rhodium aus einer wäßrigen, Rhodiumkomplexverbindungen enthaltenden Lösung ist in der US-PS 43 90 473 nicht beschrieben.

Daher besteht bei Verwendung wasserlöslicher Rhodiumkomplexverbindungen als Katalysator ein Bedarf nach einem Verfahren, Rhodium auf einfache Weise aus einer Rhodiumkomplexverbindung zurückzugewinnen.

Diese Aufgabe wird überraschenderweise gelöst durch ein Verfahren zur Rückgewinnung von Rhodium aus Rhodiumkomplexverbindungen enthaltenden wäßrigen Lösungen. Es ist dadurch gekennzeichnet, daß man der Lösung ein wasserlösliches Salz einer Carbonsäure mit 7 bis 22 Kohlenstoffatomen im Überschuß, bezogen auf Rhodium, zusetzt, die Lösung darauf bei 50 bis 200 °C mit einem Oxidationsmittel behandelt und das als in Wasser unlösliche Verbindung abgeschiedene Rhodium abtrennt.

Die erfindungsgemäße Arbeitsweise ist besonders zur Rückgewinnung von Rhodium aus Rhodiumkomplexen, die in Wasser gelöst sind, und als Katalysatorphase bei der Hydroformylierung von Olefinen verwendet werden, geeignet.

Überraschenderweise gestattet das Verfahren, das allgemein in geringer Konzentration vorliegende Rhodium mit großer Selektivität aus der wäßrigen Lösung in einer Form, die eine unmittelbare Wiederverwendung des Rhodiums als Katalysatorbestandteil zuläßt, zurückzugewinnen.

Die dem Rückgewinnungsverfahren zugeführten Lösungen enthalten den Rhodiumkomplex, überschüssigen Komplexliganden sowie dessen Abbau- und Umwandlungsprodukte in Wasser gelöst.

Der Rhodiumkomplex entspricht der allgemeinen Formel $HRh(CO)_xL_{4-x}$, wobei L für wasserlösliche Komplexliganden steht und x den Zahlen 1 bis 3 entspricht. Bei den wasserlöslichen Komplexliganden handelt es sich insbesondere um Phosphine der Formel

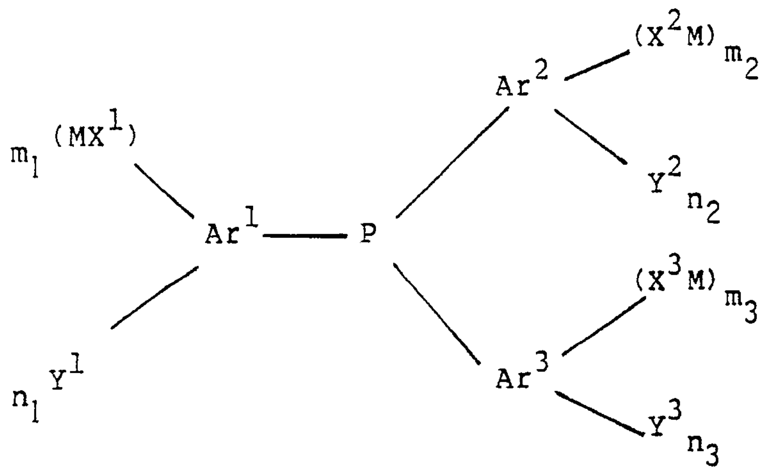

Hierin steht $Ar^1$, $Ar^2$ und $Ar^3$ jeweils für eine Phenyl-oder Naphthylgruppe, $Y^1$, $Y^2$, $Y^3$ jeweils für eine geradkettige oder verzweigte Alkylgruppe mit 1 bis 4 C-Atomen, eine Alkoxygruppe, ein Halogenatom, eine OH-, CN- $NO_2$- oder $R^1R^2N$-Gruppe, in der $R^1$ und $R^2$ jeweils eine geradkettige oder verzweigte Alkylgruppe mit 1 bis 4 C-Atomen ist; $X^1$, $X^2$, $X^3$ bedeutet jeweils einen Carboxylat-($COO^-$) und/oder Sulfonat-($SO_3$-)-Rest, $n_1$, $n_2$, $n_3$ sind gleiche oder verschiedene ganze Zahlen von 0 bis 5, M stellt ein Alkalimetallion, das Äquivalent eines Erdalkalimetall- oder Zinkions oder ein Ammonium- oder quartäres Alkylammoniumion der allgemeinen Formel $N(R^3R^4R^5R^6)^+$ dar, in der $R^3$, $R^4$, $R^5$, $R^6$ jeweils eine geradkettige oder verzweigte Alkylgruppe mit 1 bis 4 C-Atomen ist, und $m_1$, $m_2$, $m_3$ sind gleiche oder verschiedene ganze Zahlen von 0 bis 3, wobei mindestens eine Zahl $m_1$, $m_2$ oder $m_3$ gleich oder größer als 1 ist.

Der Rhodiumkomplex liegt in einer Konzentration von 10 bis 2000, insbesondere von 80 bis 800, bevorzugt von 100 bis 200 Gew.-ppm, vor. Die wäßrige Lösung enthält 0,5 bis 15, insbesondere 0,7 bis 10, bevorzugt 0,8 bis 2,0 Gew.-% wasserlöslichen Komplexliganden bezogen auf wäßrige Lösung. Daneben befinden sich in der Lösung noch Abbau- und Umwandlungsprodukte des wasserlöslichen Komplexliganden. Hierzu gehören organische Substituenten enthaltende Phosphinoxide und Phosphinsulfide, Sulfonsäuren, Phosphinsäuren, Carbonsäuren und deren Salze. Ihre Konzentration beträgt 1 bis 15, insbesondere 3 bis 12, bevorzugt 5 bis 10 Gew.-% bezogen auf wäßrige Lösung.

Der Salzrückstand, berechnet als Trockensubstanz, beträgt 1,5 bis 30, insbesondere 3,7 bis 20, bevorzugt 5,8 bis 15 Gew.-%, bezogen auf wäßrige Lösung. Unter Salzrückstand versteht man die Summe aller salzartigen Bestandteile, d.h. den Rhodiumkomplexliganden und dessen Abbau- und Umwandlungsprodukte.

Im Falle eines Hydroformylierungskatalysators weist die wäßrige Lösung insgesamt 0,15 bis 4,0, insbesondere 0,8 bis 3,0, bevorzugt 1,0 bis 1,5 Gew.-% organische Bestandteile bezogen auf wäßrige Lösung auf. Hierunter fallen eingesetztes Olefin, Aldehyde, Alkohole, Aldole, Kondensationsprodukte und gegebenenfalls Lösungsvermittler.

Den Lösungsvermittlern fällt die Aufgabe zu, die physikalischen Eigenschaften der Grenzflächen zwischen der organischen, Olefin enthaltenden, und der wäßrigen Katalysatorphase zu verändern und den Übergang der organischen Reaktanten in die Katalysatorlösung und den des wasserlöslichen Katalysatorsystems in die organische Phase zu begünstigen.

Die wäßrige, Rhodiumkomplex enthaltende Lösung wird erfindungsgemäß mit dem wasserlöslichen Salz einer Carbonsäure im Überschuß, bezogen auf Rhodium, versetzt und erhitzt. Nach Erreichen der vorgegebenen Temperatur wird die Oxidation durchgeführt. Dabei fällt das Rhodium als wasserunlösliches Rhodiumcarboxylat an, das unmittelbar abgetrennt werden kann. Man kann es jedoch auch in einem wasserunlöslichen, organischen Solvens lösen, um das Rhodiumcarboxylat leichter von der Wasserphase zu separieren.

Das erfindungsgemäße Verfahren verwendet Salze von Carbonsäuren mit 7 bis 22 Kohlenstoffatomen, insbesondere Salze von Carbonsäuren mit 8 bis 13 Kohlenstoffatomen. Hierunter fallen Salze aliphatischer, cycloaliphatischer, aromatischer und/oder araliphatischer Carbonsäuren. Gut geeignet sind Salze von Monocarbonsäuren der aliphatischen, cycloaliphatischen, aromatischen und/oder araliphatischen Reihe, insbesondere Salze verzweigter aliphatischer Monocarbonsäuren und bevorzugt Salze der 2-Ethylhexansäure, der Isononansäure (hergestellt durch Hydroformylierung von Diisobutylen und nachfolgende Oxidation des Hydroformylierungsproduktes) und/oder der Isotridecansäure (hergestellt durch Hydroformylierung von

Tetrapropylen und nachfolgende Oxidation des Hydroformylierungsproduktes). Ferner haben sich auch Salze der Phenylessigsäure und der α - und β-Naphthoesäure als brauchbar erwiesen.

Es empfiehlt sich, Alkali- und/oder Ammoniumsalze von Carbonsäuren, insbesondere Natrium- und/oder Kaliumsalze, bevorzugt Natriumsalze, zu verwenden.

Je g-Atom Rhodium werden der wäßrigen Lösung 20 bis 500, insbesondere 40 bis 300, bevorzugt 50 bis 200 mol Carbonsäuresalz zugesetzt.

Das Verhältnis Carbonsäuresalz zu Rhodium richtet sich auch nach der in der wäßrigen Lösung vorliegenden Rhodiumkonzentration. Ein niedriger Rhodiumgehalt gestattet es, das Carbonsäuresalz - entsprechend seiner Löslichkeit - in besonders hohem Überschuß zu verwenden, während höhere Rhodiumkonzentrationen zu entsprechend niedrigeren Verhältnissen Carbonsäuresalz zu Rhodium führen.

Das Salz der Carbonsäure kann der wäßrigen Lösung vor Erhitzen zugesetzt werden, es ist allerdings auch möglich, es während oder nach Erreichen der Temperatur, die für die Oxidation notwendig ist, einzubringen. Entscheidend ist lediglich, daß das Salz der Carbonsäure bereits während der Oxidation anwesend ist Eine spätere Salzzugabe führt zu nicht vorhersehbaren schlechteren Resultaten.

Ferner ist zu beachten, daß der Salzrückstand einen oberen Grenzwert nicht übersteigt. Enthält die aufzuarbeitende wäßrige Lösung mehr als 30 Gew.-% Salzrückstand, bezogen auf wäßrige Lösung, so verschlechtert sich die Rhodiumausbeute erheblich. Eine wirksame Ausübung des erfindungsgemäßen Verfahrens ist dann nicht mehr gegeben.

Die Oxidation der wäßrigen Lösungen wird bei Temperaturen von 50 bis 200, insbesondere 60 bis 160, bevorzugt 80 bis 140 °C, durchgeführt. Es ist notwendig, sie bei erhöhten Temperaturen vorzunehmen, um eine vollständige Umsetzung zu erreichen. Arbeitet man bei einer Temperatur unterhalb 50 °C, so sinken die Ausbeuten des zurückgewonnenen Rhodiums ab, und es verbleibt ein nicht unbeträchtlicher Anteil des Rhodiums in der wäßrigen Phase.

Als Oxidationsmittel werden reiner Sauerstoff, Sauerstoff enthaltende Gasgemische und insbesondere Luft verwendet.

Es ist jedoch auch möglich, andere Oxidationsmittel, wie Wasserstoffperoxid, Wasserstoffperoxid bildende Verbindungen, Hypochlorit, Chromate, Permanganate zu gebrauchen.

Besonders geeignet ist Wasserstoffperoxid.

Die Oxidation kann sowohl unter Normaldruck, als auch unter erhöhtem Druck durchgeführt werden. Geeignete Drücke sind 0,1 bis 2,0, insbesondere 0,2 bis 1 bevorzugt, 0,3 bis 0,7 MPa.

Der pH-Wert der wäßrigen Lösung soll 4 bis 8, insbesondere 5,0 bis 7,5, bevorzugt 5,5 bis 7,0, betragen. Da im Falle der Hydroformylierung in der wäßrigen Phase stets noch untergeordnete Mengen Aldehyde, aus denen sich während der Oxidation Carbonsäuren bilden, vorhanden sind, empfiehlt es sich, den pH-Wert während der Oxidation nachzumessen und gegebenenfalls auf den erforderlichen Bereich neu einzustellen.

Will man die Anwesenheit der aus den Aldehyden gebildeten Carbonsäuren gänzlich vermeiden, so unterzieht man die aufzuarbeitende wäßrige Lösung einer Destillation oder treibt, indem man beispielsweise überhitzten Wasserdampf hindurchleitet, die organischen Produkte über, ehe man oxidiert.

Infolge der Oxidation wird nicht nur der im Überschuß vorhandene Komplexligand, sondern auch der Rhodiumkomplex selbst angegriffen, wobei der Ligand in eine Form, die sich nicht mehr zur Komplexbildung eignet, umgewandelt wird.

Aus den vorstehend genannten Phosphinen bilden sich die entsprechenden Phosphinoxide, und der ursprünglich vorhandene Rhodiumkomplex fällt auseinander.

Im Verlauf der Oxidation bilden sich wasserunlösliche Rhodiumverbindungen (Rhodiumcarboxylate) und scheiden sich als ölige Schicht oder in Tropfenform ab. Die Trennung kann durch einfache Phasenseparation erfolgen, wobei die untere, wäßrige Phase entfernt wird.

Es ist jedoch hilfreich, die Rhodiumrückgewinnung durch Zusatz wasserunlöslicher, organischer Solventien zu unterstützen, die in der Lage sind, die wasserunlösliche Rhodiumverbindung zu lösen.

Der Zusatz des organischen Lösungsmittels kann bereits vor oder während der Oxidation erfolgen. Besonders bewährt hat es sich, das Lösungsmittel dem Reaktionsgemisch nach der Oxidation bei 10 bis 100, insbesondere 20 bis 70, bevorzugt 40 bis 60 °C, zuzugeben.

Geeignete organische Lösungsmittel sind Benzol, Toluol, Xylol, Cyclohexan, aliphatische Carbonsäuren und Carbonsäureester, aliphatische und cycloaliphatische Ketone mit 5 bis 10 Kohlenstoffatomen. Besonders geeignet sind Toluol und Xylol, bevorzugt wird Toluol verwendet.

Die obere, das organische Lösungsmittel und die wasserunlösliche Rhodiumverbindung enthaltende Phase wird von der unteren, wäßrigen Lösung abgetrennt. Die Phasenseparation wird bei 10 bis 100, insbesondere 20 bis 70, bevorzugt 40 bis 60 °C durchgeführt.

Zur Vervollständigung der Rhodiumrückgewinnung extrahiert man die abgetrennte wäßrige Phase - falls

erforderlich - nochmals mit frischem organischen Lösungsmittel und trennt die Phasen. Dieser Vorgang kann mehrfach wiederholt werden. In der Regel genügt es, die wäßrige Phase ein- bis dreimal zu extrahieren.

Die organischen Phasen werden vereint und können ohne weitere Nachbehandlung unmittelbar als Katalysatorbestandteil gebraucht werden. Es ist auch möglich, das in dem organischen Lösungsmittel vorliegende Rhodiumcarboxylat durch Extraktion mit wäßrigen, Phosphinligand enthaltenden Lösungen zu extrahieren und diesen Extrakt als Katalysatorlösung zu verwenden.

Durch das erfindungsgemäße Verfahren können etwa 90 bis 95 % des ursprünglich vorhandenen Rhodiums aus der wäßrigen Lösung abgetrennt werden. Das restliche Rhodium verbleibt in der Wasserphase und kann gesondert zurückgewonnen werden.

Die nachfolgenden Beispiele sollen die Erfindung näher erläutern, ohne sie zu beschränken.

Beispiel 1

300 g der Lösung A (s. Tabelle 1) und 18,55 g einer wäßrigen Na-2-ethylhexanoat-Lösung (65,8 Gew.-%Na-salz, bezogen auf die Lösung) werden in einem Glasautoklaven unter Rühren bei 100 °C und einem Druck von 0,4 bis 0,45 MPa durch Einleiten von 150 Nl Luft oxidiert. Die Umsetzung ist nach 2 Stunden beendet, der Inhalt des Autoklaven wird abgekühlt. Es bildet sich eine ölige Schicht, die durch Extraktion mit 60 g und nochmaliger Extraktion mit 50 g Toluol abgetrennt wird. In der wäßrigen Lösung verbleiben 8,2 % der ursprünglich vorhandenen Rhodiummenge.

Der Versuch wird unter den gleichen Bedingungen wiederholt. Die wäßrige Lösung enthält noch 7,5 % der ursprünglich vorhandenen Rhodiummenge.

Beispiel 2

Beispiel 1 wird wiederholt mit dem Unterschied, daß die wäßrige Na-2-ethylhexanoat-Lösung erst nach Abschluß der Oxidation bei 100 °C hinzugefügt wird. Die wäßrige Lösung wird anschließend wiederum abgekühlt und zweimal mit Toluol extrahiert. In der wäßrigen Lösung verbleiben noch 75,1 Gew.-% der ursprünglich vorhandenen Rhodiummenge.

Beispiel 3

300 g der in Tabelle 1 ausgewiesenen Lösung B werden mit 8,0 g einer wäßrigen Na-2-ethylhexanoat-Lösung (65,8 Gew.-% Na-Salz bezogen auf Lösung) und 5,0 g 2-Ethylhexansäure in einen Glasautoklaven (Volumen: 1 l) gefüllt. Unter Rühren wird auf 100 °C aufgeheizt und die Oxidation nach Erreichen dieser Solltemperatur durch Zugabe von Wasserstoffperoxid herbeigeführt. Man gibt 10 g Wasserstoffperoxid (30 Gew.-%-ig) über einen Zeitraum von 5 Minuten zu und läßt das Gemisch unter dem sich bildenden Eigendruck reagieren. Die Oxidation ist nach 30 Minuten beendet.

Der Inhalt des Glasautoklaven wird abgekühlt und die wäßrige Lösung zunächst mit 60 g Toluol versetzt, ausgeschüttelt und nach Abtrennung der Toluolphase erneut mit 50 g Toluol extrahiert. Nach Separation der Toluolphase verbleiben in der wäßrigen Lösung 6,2 % des ursprünglich vorhandenen Rhodiums.

Beispiel 4

Beispiel 3 wird wiederholt, jedoch wird anstelle von 8,0 g wäßriger Na-2-ethylhexanoat-Lösung 3,8 g Na-butyrat und anstelle von 5,0 g 2-Ethylhexansäure 3,1 g Buttersäure verwendet. Nach Extraktion mit Toluol - wie in Beispiel 3 angegeben - verbleiben in der wäßrigen Lösung noch 90,7 % des ursprünglich vorhandenen Rhodiums.

Beispiel 5

300 g der in Beispiel 3 verwendeten wäßrigen Lösung B (Zusammensetzung s. Tabelle 1) werden mit 8,0 g einer Na-2-ethylhexanoat-Lösung (65,8 Gew.-% Na-Salz bezogen auf Lösung) in einen Glasautoklaven (Volumen: 1 l) gefüllt. Unter Rühren wird auf 100 °C aufgeheizt und die Oxidation nach Erreichen dieser Solltemperatur durch Einleiten von reinem Sauerstoff bei einem Druck von 0,5 MPa durchgeführt. Die Oxidation wird nach 4 Stunden beendet und es wird, - wie in Beispiel 3 angegeben-, abgekühlt und mit Toluol extrahiert. In der wäßrigen Lösung verbleiben 4,8 % des ursprünglich vorhandenen Rhodiums.

Ein weiterer Versuch liefert unter den gleichen Bedingungen eine wäßrige Lösung, in der 6.5 % des ursprünglich vorhandenen Rhodiums enthalten sind.

Beispiel 6

Beispiel 5 wird wiederholt. Abweichend hiervon wird zusätzlich zur Na-2-ethylhexanoat-Lösung 5 g 2-Ethylhexansäure zugegeben. Die Oxidation wird ebenfalls bei 100°C durchgeführt, allerdings beträgt die Dauer 2 Stunden. Anschließend wird - wie in Beispiel 3 angegeben - abgekühlt und mit Toluol extrahiert. In der wäßrigen Lösung verbleiben 4,7 % des ursprünglich vorhandenen Rhodiums.

Ein weiterer Versuch liefert unter den gleichen Bedingungen - allerdings bei einer Oxidationsdauer von 1 Stunde - eine wäßrige Lösung, in der 6,0 % des ursprünglich vorhandenen Rhodiums enthalten sind.

Beispiel 7 a bis i

300 g der in Tabelle 1 ausgewiesenen Lösung C werden mit den in Tabelle 2 genannten Mengen Carbonsäure und NaOH versetzt und in einen Glasautoklaven (Volumen: 1 l) gefüllt. Unter Rühren wird auf 100°C aufgeheizt und die Oxidation nach Erreichen dieser Solltemperatur durch Einleiten von Luft bei einem Druck von 0,4 MPa durchgeführt. Die zugesetzte Menge Luft ist ebenfalls Tabelle 2 zu entnehmen. Die Oxidation ist nach 4 Stunden beendet.

Der Inhalt des Glasautoklaven wird - wie in Beispiel 1 angegeben - aufgearbeitet.
Die in der wäßrigen Lösung nach Extraktion verbleibende Rhodiummenge ist Tabelle 2 (Rhodium im Abwasser) zu entnehmen.

Beispiel 8 a bis g

300 g der in Tabelle 1 ausgewiesenen Lösung D werden mit den in Tabelle 3 genannten Mengen Natrium-2-ethylhexanoat (65,8 Gew.-% Na-Salz, bezogen auf Lösung) versetzt und in einen Glasautoklaven (Volumen 1 l) gefüllt. Durch Zugabe von NaOH oder $H_2SO_4$ wird der in Tabelle 3 aufgeführte pH-Wert eingestellt und während der Oxidation beibehalten. Die Lösung wird unter Rühren bei 100°C und einem Druck von 0,45 MPa durch Einleiten von 150 Nl Luft oxidiert. Die Umsetzung ist nach 2 Stunden beendet. Es wird - wie in Beispiel 1 angegeben - eine ölige Schicht gebildet und durch Extraktion mit Toluol abgetrennt.

Die in der wäßrigen Lösung nach Extraktion verbleibende Rhodiummenge ist Tabelle 3 zu entnehmen.

Beispiel 9

300 g der in Tabelle 1 ausgewiesenen Lösung D werden destilliert, um noch vorhandene organische Produkte zu entfernen. Die Destillation erfolgt unter Normaldruck. Bei einer Kopftemperatur von 100°C werden 78,3 g Produkt (entsprechend 26,1 Gew.-% bezogen auf wäßrige Lösung) abgetrennt.

Der verbleibende Destillationsrückstand wird mit destilliertem Wasser auf 300 g ergänzt und - wie in Beispiel 8 a bis g beschrieben - aufgearbeitet.

Tabelle 3 enthält Angaben bezüglich pH-Wert, Menge Natrium-2-ethylhexanoat (als 65,8 Gew.-% Na-Salz, bezogen auf Lösung) und Rhodiummenge in Abwasser.

Tabelle 1

Bestandteile in Gew.-%; Rh in ppm

| Bestandteile | Lösung A | Lösung B | Lösung C | Lösung D |
|---|---|---|---|---|
| TPPTS | 2,80 | 0,22 | 3,13 | 1,70 |
| TPPOTS | 2,45 | 1,72 | 2,0 | 1,73 |
| TPPSTS | - | 0,03 | 0,02 | - |
| TPPDS | 0,14 | < 0,01 | 0,2 | 0,04 |
| TPPODS | 0,50 | 0,48 | 0,42 | 0,38 |
| TPPSDS | 0,02 | - | 0,02 | 0,03 |
| BSNS | 0,97 | 0,92 | 0,55 | 0,55 |
| restl. Salze*) | 5,12 | 7,19 | 2,33 | 4,23 |
| Rhodium | 135 ppm | 165 ppm | 174 ppm | 124 ppm |

*) als Rückstand (Trockensubstanz) durch Flashdestillation (95°C/15 Torr (2,25 kPa)) ermittelt:

In der obigen Tabelle bedeuten:

TPPTS : $Na_3$-Triphenylphosphintrisulfonat
TPPOTS : $Na_3$-Triphenylphosphinoxidtrisulfonat
TPPSTS : $Na_3$-Triphenylphosphinsulfidtrisulfonat
TPPDS : $Na_2$-Triphenylphosphindisulfonat
TPPODS : $Na_2$-Triphenylphosphinoxiddisulfonat
TPPSDS : $Na_2$-Triphenylphosphinsulfiddisulfonat
BSNS : Na-Benzolsulfonat

Tabelle 2

| Beispiel Nr. | Carbonsäure | Lauge | Luftmenge NL | Rhodium im Abwasser % |
|---|---|---|---|---|
| 7 a | 15,4 g n-$C_5$-Säure | 3,0 g NaOH | 200 | 73,2 |
| 7 b | 15,4 g n-$C_5$-Säure | 3,0 g NaOH | 237 | 66,1 |
| 7 c | 19,53 g n-$C_7$-Säure | 3,0 g NaOH | 248 | 31,2 |
| 7 d | 25,84 g n-$C_{10}$-Säure | 3,0 g NaOH | 206 | 10,3 |
| 7 e | 14,51 g 2-Ethyl-hexansäure | 2,636 g NaOH | 190 | 3,6 |
| 7 f | 19,51 g 2-Ethyl-hexansäure | 2,636 g NaOH | 200 | 4,8 |
| 7 g | 17,0 g Iso-$C_9$-Säure 1) | 3,05 g NaOH | 266 | 3,5 |
| 7 h | 21,35 g Iso-$C_{13}$-Säure 2) | 3,05 g NaOH | 182 | 5,7 |
| 7 i | 26,71 g Iso-$C_{18}$-Säure 3) | 3,05 g NaOH | 160 | 55,6 |

1) Hergestellt durch Hydroformylierung von Diisobutylen und nachfolgende Oxidation.
2) Hergestellt durch Hydroformylierung von Tetrapropylen und nachfolgende Oxidation.
3) Hauptkomponente 2,2,4,8,10,10-Hexamethylundecancarbonsäure-7

Tabelle 3

| Beispiel-Nr. | pH-Wert | Na-2-ethylhexanoat (g) | Rhodium im Abwasser (%) |
|---|---|---|---|
| 8 a | 3,3 | 14,45 | 26 |
| 8 b | 4,5 | 14,45 | 13,6 |
| 8 c | 5,8 | 14,45 | 6,2 |
| 8 d | 6,2 | 14,45 | 6,1 |
| 8 e | 6,6 | 13,8 | 7,7 |
| 8 f | 8,7 | 14,45 | 33,5 |
| 8 g | 10,3 | 14,45 | 36,8 |
| 9 | 6,9 | 13,8 | 6,7 |

## Patentansprüche

1. Verfahren zur Rückgewinnung von Rhodium aus Rhodiumkomplexverbindungen enthaltenden wäßrigen Lösungen, dadurch gekennzeichnet, daß man der Lösung ein wasserlösliches Salz einer Carbonsäure mit 7 bis 22 Kohlenstoffatomen im Überschuß, bezogen auf Rhodium zusetzt; die Lösung darauf bei 50 bis 200 °C mit einem Oxidationsmittel behandelt und das als in Wasser unlösliche Verbindung abgeschiedene Rhodium abtrennt.

2. Verfahren nach Anspruch 1, dadurch gekennzeichnet, daß man der wäßrigen Lösung das Salz einer gesättigten, geradkettigen oder verzweigten Monocarbonsäure zusetzt.

3. Verfahren nach einem oder mehreren der Ansprüche 1 und 2, dadurch gekennzeichnet, daß die Carbonsäure 8 bis 13 Kohlenstoffatome enthält.

4. Verfahren nach einem oder mehreren der Ansprüche 1 bis 3, dadurch gekennzeichnet, daß das Salz der Carbonsäure ein Alkalisalz ist.

5. Verfahren nach einem oder mehreren der Ansprüche 1 bis 4, dadurch gekennzeichnet, daß der wäßrigen Lösung 20 bis 500 mol Carbonsäuresalz je g-Atom Rhodium zugesetzt werden.

6. Verfahren nach einem oder mehreren der Ansprüche 1 bis 5, dadurch gekennzeichnet, daß als Oxidationsmittel Sauerstoff oder ein Sauerstoff enthaltendes Gas verwendet wird.

7. Verfahren nach einem oder mehreren der Ansprüche 1 bis 6, dadurch gekennzeichnet, daß als Oxidationsmittel Luft verwendet wird.

8. Verfahren nach einem oder mehreren der Ansprüche 1 bis 7, dadurch gekennzeichnet, daß der pH-Wert der wäßrigen Lösung 4 bis 8 beträgt.

9. Verfahren nach einem oder mehreren der Ansprüche 1 bis 8, dadurch gekennzeichnet, daß die wäßrige Lösung unter einem Druck von 0,1 bis 2,0 MPa mit dem Oxidationsmittel behandelt wird.

10. Verfahren nach einem oder mehreren der Ansprüche 1 bis 9, dadurch gekennzeichnet, daß die in Wasser unlösliche, abgeschiedene Rhodiumverbindung mit einem wasserunlöslichen organischen Lösungsmittel extrahiert wird.

11. Verfahren nach Anspruch 10, dadurch gekennzeichnet, daß das organische Lösungsmittel Toluol ist.

**Claims**

1. A process for the recovery of rhodium from aqueous solutions containing rhodium complex compounds, characterised in that a water-soluble salt of a carboxylic acid with 7 to 22 carbon atoms is added to the solution in excess related to the rhodium, the solution is then treated with an oxidant at 50 to 200 °C and the rhodium, which separates as a water-insoluble compound, removed.

2. A process according to claim 1, characterised in that the salt of a saturated, straight-chain or branched monocarboxylic acid is added to the aqueous solution.

3. A process according to one or both of claims 1 and 2, characterised in that the carboxylic acid contains 8 to 13 carbon atoms.

4. A process according to one or several of claims 1 to 3, characterised in that the salt of the carboxylic acid is an alkali metal salt.

5. A process according to one or several of claims 1 to 4, characterised in that 20 to 500 mol carboxylic acid salt are added to the aqueous solution per g-atom rhodium.

6. A process according to one or several of claims 1 to 5, characterised in that oxygen or an oxygen-containing gas is used as an oxidant.

7. A process according to one or several of claims 1 to 6, characterised in that air is used as an oxidant.

8. A process according to one or several of claims 1 to 7, characterised in that the pH value of the aqueous solution is 4 to 8.

9. A process according to one or several of claims 1 to 8, characterised in that the aqueous solution is treated with an oxidant at a pressure of 0.1 to 2.0 MPa.

10. A process according to one or several of claims 1 to 9, characterised in that the separated water-insoluble rhodium compound is extracted with a water-insoluble organic solvent.

11. A process according to claim 10, characterised in that the organic solvent is toluene.

**Revendications**

1. Procédé pour récupérer le rhodium contenu à l'état de complexes dans des solutions aqueuses, caractérisé en ce que l'on ajoute aux solutions un sel hydrosoluble d'un acide carboxylique en $C_7$-$C_{22}$ en excès par rapport au rhodium ; on traite ensuite les solutions à des températures de 50 à 200°C par un agent oxydant et on sépare le rhodium qui a précipité à l'état de composé insoluble dans l'eau.

2. Procédé selon la revendication 1, caractérisé en ce que l'on ajoute à la solution aqueuse le sel d'un acide monocarboxylique saturé à chaîne droite ou ramifiée.

3. Procédé selon une ou plusieurs des revendications 1 et 2, caractérisé en ce que l'acide carboxylique contient de 8 à 13 atomes de carbone.

4. Procédé selon une ou plusieurs des revendications 1 à 3, caractérisé en ce que le sel d'acide carboxylique est un sel alcalin.

5. Procédé selon une ou plusieurs des revendications 1 à 4, caractérisé en ce que l'on ajoute à la solution aqueuse de 20 à 500 mol du sel d'acide carboxylique par atome-grame de rhodium.

6. Procédé selon une ou plusieurs des revendications 1 à 5, caractérisé en ce que l'on utilise en tant qu'agent oxydant l'oxygène ou un gaz contenant de l'oxygène.

7. Procédé selon une ou plusieurs des revendications 1 à 6, caractérisé en ce que l'on utilise l'air en tant qu'agent oxydant.

8. Procédé selon une ou plusieurs des revendications 1 à 7, caractérisé en ce que le pH de la solution aqueuse est de 4 à 8.

9. Procédé selon une ou plusieurs des revendications 1 à 8, caractérisé en ce que la solution aqueuse est traitée par l'agent oxydant sous une pression de 0,1 à 2,0 MPa.

10. Procédé selon une ou plusieurs des revendications 1 à 9, caractérisé en ce que le composé du rhodium qui a précipité à l'état insoluble dans l'eau est extrait par un solvant organique insoluble dans l'eau.

11. Procédé selon la revendication 10, caractérisé en ce que le solvant organique est le toluène.